# EUROPEAN PATENT SPECIFICATION

(11) **EP 2 268 627 B1**
(45) Date of publication and mention of the grant of the patent: **14.12.2011**
(21) Application number: 09738065.3
(22) Date of filing: 24.04.2009
(51) Int. Cl.: C07D 307/60

(54) **PAPER SIZING ADDITIVES, THEIR PREPARATION PROCESS, AND THEIR USE**
PAPIERLEIMUNGSZUSÄTZE, DEREN HERSTELLUNGSVERFAHREN UND VERWENDUNG
ADDITIFS D'ENCOLLAGE DE PAPIER, PROCÉDÉ DE PRÉPARATION ET UTILISATION DE CES DERNIERS

(30) Priority: 29.04.2008 EP 08155398; 15.05.2008 US 53582 P
(43) Date of publication of application: 05.01.2011
(73) Proprietor: Akzo Nobel N.V., 6824 BM Arnhem (NL)
(72) Inventor: DE JONG, Aaldert, Johannes, NL-3781 XN Voorthuizen (NL); WERNER, Martin, 50374 Erftstadt (DE); STRUCK, Oliver, 91239 Henfenfeld (DE)
(74) Representative: Alferink, Petrus J.T.
(86) International application number: PCT/EP2009/054931
(87) International publication number: WO 2009/133021

(56) References cited:
- EP-A- 0 190 352
- EP-A- 1 424 322
- WO-A-00/79050
- US-A- 3 405 196
- US-A- 3 928 485
- US-A- 5 021 169
- S. H. NAHM, H. N. CHENG: "Transition-state geometry and stereochemistry of the ene reaction between olefins and maleic anhydride" J. ORG. CHEM, vol. 51, 1986, pages 5093-5100, XP002502657

## Description

The present invention relates to novel paper sizing additives, to a process to prepare alkenyl-substituted cyclic anhydrides by isomerization of alpha-olefins followed by reaction with a cyclic anhydride of an unsaturated dicarboxylic acid, and to their use in conjunction with paper and paperboard production. Furthermore, the present invention relates to a process for the preparation of sizing agents for paper from a cyclic anhydride of an unsaturated dicarboxylic acid and internal olefins isomerized using an alkali metal catalyst. Alkenyl-substituted cyclic anhydrides are used extensively in the papermaking industry as a paper sizing additive for improving the properties of paper, including fine paper, recycled linerboard, and gypsum board. Alkenyl succinic anhydride (ASA) compounds are the most commonly used alkenyl-substituted cyclic anhydrides. ASA compounds have reactive functional groups that are believed to covalently bond to cellulose fibres, and hydrophobic tails that are oriented away from the fibres. The nature and orientation of these hydrophobic tails causes the fibres to repel water. Commercial sizing agents based on ASA compounds are typically prepared from maleic anhydride and one or more appropriate alpha- and/or internal olefins, for example C₁₆ internal olefins and/or C₁₈ internal olefins.

Internal olefins are usually made from linear alpha-olefins by isomerizing the olefin double bond from a terminal to an internal position. Linear alpha-olefins have a structure as indicated in Figure 1: 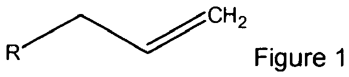 wherein R is an aliphatic hydrocarbon group.

However, substantially pure linear alpha-olefins having the appropriate chain length required for ASA compounds are rather rare on the market and relatively expensive. In practice, a substantial portion of the alpha-olefins on the market will have the structure indicated in Figure 2, 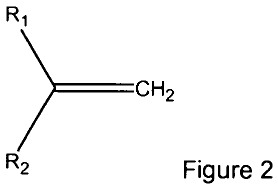 with R₁ and R₂ groups being aliphatic hydrocarbon groups, the so-called vinylidene olefins. The vinylidene content and the degree of branching in alpha-olefins are strongly dependent on the ethylene oligomerization processes run by the different alpha-olefin suppliers. These production processes differ substantially from each other (see for example Industrial Organic Chemicals: Starting Materials and Intermediates, Wiley-VCH Verlag GmbH, 1999, Volume 5, Chapter 2.1 entitled Monoolefins, pages 2870-2873).

Generally, alpha-olefin mixtures comprising at least 10% by weight of vinylidene olefins are more readily available (they can for example be obtained from Ineos and Nizhnekamskneftekhim), are cheaper, and are more easily accessible as a raw material than substantially pure linear alpha-olefins. This is to a large extent because large quantities of the available linear alpha-olefins are sold to the ASA industry either directly or after an isomerization step. Hence, it would be economically attractive if it were to be possible to make alkenyl-substituted cyclic anhydride compounds with an acceptable to good performance as paper additives based on alpha-olefins comprising a substantial amount of vinylidene olefins.

US 6,231,659 discloses alkenyl succinic anhydrides for use as paper sizing agents which are a mixture of alkenyl succinic anhydrides in which the alkenyl groups have in the range of about 6 to about 40 carbon atoms, and in which at least 97 wt% of the alkenyl groups are bifurcated on the alpha carbon atom into two branches neither of which has less than two carbon atoms. These alkenyl succinic anhydrides are prepared from maleic anhydride and made up of linear or substantially linear internal olefins or they are made up of linear or substantially linear olefins in admixture with vinylidene olefins. They are also obtained by isomerization of the corresponding alpha-olefins using iron pentacarbonyl as the catalyst.

Disadvantages of this homogeneous process are that the isomerization step can only be performed at relatively high temperatures and that the obtained olefins have to be cleaned by distillation to remove undesired catalyst residues and colouring (caused by the catalyst and catalyst decomposition products) before they can be reacted with maleic anhydride to form ASA compounds. Furthermore, the performance of ASA compounds based on alpha-olefins comprising vinylidene olefins which have been isomerized using iron pentacarbonyl as the catalyst when used as paper sizing agents is significantly lower than if ASA compounds based on isomerized linear alpha-olefins are used.

EP 1 424 322 discloses a low molecular weight branched alkenyl succinic acid derivative obtainable by reacting a low molecular weight polyisobutylene having from about 8 to 32 carbon atoms, and wherein at least about 50% of the olefinic bonds of the polyisobutene comprise methylvinylidene isomer plus tri-substituted isomer, with an unsaturated acidic reagent and method of making the same, resulting in alkenyl succinic acid derivative isomers.

WO00/79050 discloses paper sizing agents which are mixtures of alkenyl succinic anhydrides in which the alkenyl groups have in the range of 6 to 40 carbon atoms, and in which at least 97 wt% of the alkenyl groups are bifurcated on the alpha carbon atom into two branches neither of which has less than two carbon atoms.

It is an object of the present invention to provide an improved preparation process for alkenyl-substituted cyclic anhydride compounds. Furthermore, it is an object of the present invention to provide a preparation process for alkenyl-substituted cyclic anhydride compounds wherein alpha-olefins having a relatively high percentage of vinylidene isomers can be used as starting material and wherein the resulting alkenyl-substituted cyclic anhydride compounds have an acceptable to good paper sizing performance.

These objectives are realized with the preparation process for an alkenyl-substituted cyclic anhydride compound according to the invention, which comprises the steps of
(i) subjecting one or more olefinically unsaturated C₆ - C₂₈ hydrocarbons of which at least 30% by weight is alpha-olefin to a double bond isomerization step by contacting them with a catalyst comprising an alkali metal on a carrier, and
(ii) reacting the resulting isomerized olefinic C₆ - C₂₈ hydrocarbons with a cyclic anhydride of an unsaturated dicarboxylic acid to form the alkenyl-substituted cyclic anhydride compound.

The compounds that can be used as starting material in the process according to the present invention are olefinically unsaturated hydrocarbons with a chain length of from 6 to 28 carbon atoms. The olefinicallly unsaturated hydrocarbon can also be any mixture of C₆ - C₂₈ olefinically unsaturated hydrocarbons. Preferably, C₁₆ - C₂₄ olefinically unsaturated hydrocarbons or mixtures thereof are used. Most preferably, use is made of C₁₆ or C₁₈ olefinically unsaturated hydrocarbons or a mixture of olefinically unsaturated C₁₆ and C₁₈ hydrocarbons.

At least 30% by weight of these olefinically unsaturated hydrocarbons are alpha-olefins. Preferably, at least 50%, more preferably at least 75%, even more preferably at least 90% and most preferably at least 95% by weight of these olefinically unsaturated hydrocarbons are alpha-olefins. Advantages of the process according to the present invention are that the obtained olefins do not necessarily have to be cleaned by distillation before they can be reacted with a cyclic anhydride of an unsaturated dicarboxylic acid, such as maleic anhydride, to form an alkenyl-substituted cyclic anhydride compound, because there is significantly less colouring or even no colouring at all when using the heterogeneous catalyst according to the present invention compared to the previously described prior art processes wherein a homogeneous catalyst is used. Furthermore, in contrast to the well-known acid-catalyzed isomerizations wherein rearrangements of the carbon skeleton will take place, which leads to significant branching, no such branching occurs when using the process according to the present invention. Preferably, at least 15%, preferably at least 25%, and most preferably at least 35% by weight of the said alpha-olefins are vinylidene isomers.

The catalyst used in the process according to the present invention comprising an alkali metal on a carrier and a method for its preparation is for example described in GB 1416317, GB 1,492,059, US 2,952,719, and US 3,897,509. The alkali metal is selected from the group consisting of lithium, sodium, potassium, rubidium, cesium, and mixtures thereof. Of these alkali metals, the more plentiful and less expensive sodium and potassium are preferred, either alone or in admixture with one another.

The alkali metal component of the isomerization catalyst may be supported on a wide variety of carriers. The carrier must be anhydrous, i.e. free or substantially free of water. This can be achieved by precalcination of the carrier. This precalcination is usually carried out at a relatively high temperature, for example 400 - 700°C, and for a time sufficient to effect substantial removal of adsorbed or combined water from the carrier. The carrier must be inert, i.e. the carrier should not react chemically with the alkali metal. Examples of carriers that can be used are aluminium oxide (gamma-, eta-, or theta-), silicon oxide, magnesium oxide, silicon oxide-aluminium oxide, silicon oxide-aluminium oxide-magnesium oxide, titanium oxide, zirconium oxide, bauxite, clays, pumice, activated carbon, and molecular sieves. More preferably, it is an aluminium oxide carrier (also commonly referred to as alumina). Preferably, the carrier has a specific surface area larger than 25 m²/g, more preferably larger than 100 m²/g (as determined by the BET method). Most preferred is gamma-aluminium oxide.

The alkali metal may be deposited on the carrier in any suitable manner. One manner which has been found to be particularly advantageous is vaporization of the alkali metal and passage of the vapours over the carrier. This process is carried out at relatively low temperatures. Sodium, for example, melts at about 97°C and in impregnating a selected carrier with sodium it is preferred to carry out the impregnation or disposal of the sodium thereon at temperatures in the range of 100 - 150°C. This can be accomplished, for example, by melting sodium and dropping the molten sodium on the carrier or by passage of a stream of inert gas such as nitrogen or argon through the molten sodium and over a bed of the selected carrier disposed in a separate zone maintained at the desired temperature with cooling or heating means connected therewith. Potassium melts at about 62°C, and thus the impregnation of a selected carrier with potassium can be carried out at even lower temperatures.

Another method for preparing the catalyst comprising an alkali metal on a carrier according to the present invention takes the form of stirring the alkali metal together with the carrier at a temperature above the melting temperature of said alkali metal under an inert atmosphere.

Catalysts with improved stability towards air and water can be obtained by using particular additives in the preparation method. More specifically, the catalyst is preferably prepared using carbonates, sulphates, hydroxides or oxides of the above-mentioned alkali metals as additives. Alkali metal halides are not recommended for use as additives, because the carrier is generally not capable of withstanding the temperature (about 800°C) at which these compounds react with sodium or lithium. Temperatures which are very suitable if carbonates are used as additives are those between 160 and 200°C. If a hydroxide is used as additive, the catalyst is prepared by heating an alkali metal, an alkali metal hydroxide, and alumina at a temperature higher than the melting point of the alkali metal. The metal can also be used in the form of an alloy consisting of two or more kinds of alkali metals. A typical example of such an alloy is sodium-potassium alloy. Examples of the alkali metal hydroxide are hydroxides of lithium, sodium, potassium, rubidium, and other metals in Group I of the Periodic Table. One or more kinds of these hydroxides can be used. The alkali metal and the alkali metal hydroxide to be employed may be, for instance, lithium and lithium hydroxide, sodium and sodium hydroxide, potassium and potassium hydroxide, or rubidium and rubidium hydroxide, sodium and potassium hydroxide, or lithium and potassium hydroxide. It is also possible to start from solutions containing compounds which during heating are converted into other alkali metal compounds. For instance, bicarbonates or formates, both of which are converted into carbonates during heating, can be used. However, formates are less preferred.

In a preferred embodiment of the present invention, the catalyst is prepared using an additive selected from the group consisting of Na₂CO₃, K₂CO₃, NaOH, KOH, NaHCO₃, and KHCO₃. Most preferably, Na₂CO₃, K₂CO₃, NaOH, or KOH is used.

The shape of the catalyst particles is not crucial; the catalyst can be used in the form of powders, flakes, spheres, pellets, rings, extrudates or in any other suitable form. The catalyst particles may be used in a wide range of dimensions, for instance, as pellets with a diameter of 1-5 mm or as powders whose particles have a grain size of 15-35 mesh (largest diameter of approx 13-0.5 mm), 30-80 mesh (largest diameter of approx. 0.595-0.177 mm) or 100-325 mesh (largest diameter of 0.15-0.04 mm). In general, the migration of the double bonds proceeds faster as the catalyst particles are smaller.

If an additive other than an alkali metal hydroxide is used for the preparation of the catalyst, the alkali metal is preferably present in the catalyst in an amount of more than 1% by weight, more preferably more than 1.5% by weight, and most preferably more than 2% by weight, based on the weight of the carrier. Preferably, the alkali metal is present in an amount of not more than 70% by weight, more preferably not more than 30% by weight, and most preferably not more than 15% by weight, based on the weight of the carrier. If alkali metal hydroxides are used as additives in the preparation, the alkali metal is preferably present in the catalyst in an amount of more than 1 mol%, more preferably more than 2 mol%, and most preferably more than 3 mol%, relative to the amount of additive. Preferably, the alkali metal is present in an amount of not more than 100 mol%, more preferably not more than 40 mol% and most preferably not more than 20 mol%, relative to the amount of additive. The additive is preferably used in an amount of at least 1% by weight, more preferably at least 1.5% by weight, and most preferably at least 2% by weight, based on the weight of the carrier. The additive is preferably used in an amount of at most 100% by weight, more preferably at most 70% by weight, and most preferably at most 30% by weight, based on the weight of the carrier.

For more details on how to prepare the alkali metal catalyst suitable for use in the process of the present invention see GB 1416317, GB 1,492,059, US 2,952,719, and US 3,897,509.

Step (i) of the process according to the invention, the isomerization step of the C₆ - C₂₈ alpha-olefins, is preferably carried out in an inert atmosphere at temperatures of between 10 - 200°C, more preferably at a temperature of between 15 and 100°C, and most preferably at a temperature between 20 and 60°C. The isomerization step can be carried out in the liquid phase or in the vapour phase. Preferably, the isomerization is carried out at atmospheric pressure. The isomerized olefins obtained from the step (i) can be used in step (ii) without purification. The alpha-olefins can be contacted with the catalyst according to the present invention in any manner known in the art. The reaction can for example be carried out as a batch reaction or by directing an olefin flow through a packed bed reactor.

The amount of catalyst used relative to the total amount of olefin to be isomerized is preferably at least 1 % by weight, more preferably at least 2% by weight, and most preferably at least 4% by weight. Preferably, not more than 50% by weight, more preferably not more than 40% by weight, and most preferably not more than 20% by weight of catalyst is employed, relative to the amount of olefin to be isomerized.

In step (ii) of the process according to the present invention, the olefinic C₆ - C₂₈ hydrocarbons resulting from step (i) are reacted with a cyclic anhydride of an unsaturated dicarboxylic acid, such as maleic anhydride, at a temperature and for a time sufficient to provide the alkenyl-substituted cyclic anhydride compound as indicated in Figure 3, 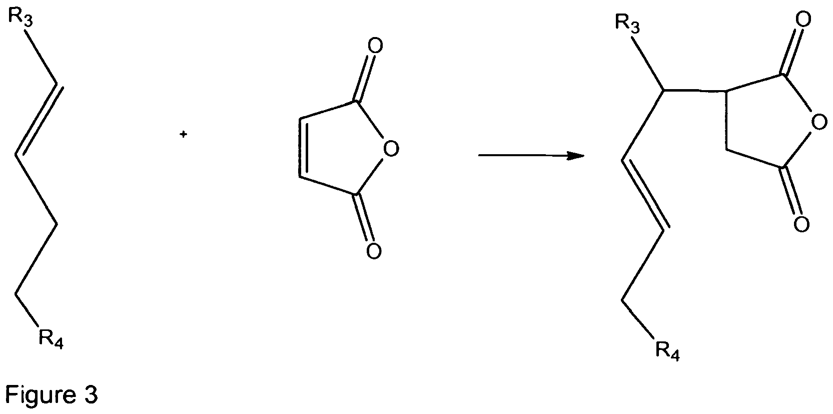 with R₃ and R₄ groups being aliphatic hydrocarbon groups. This is a so-called ene reaction. The ene reaction requires a relatively high temperature, since its activation energy is high, i.e. approx. 20 kcal/mol. The reaction speed of the reaction also increases strongly as a function of the temperature, and thus it is preferable to use a relatively high reaction temperature in order for the product to form at a reaction speed which is at least satisfactory. The reaction can be performed in any manner known in the art, for example as described in WO 97/30039.

In the process according to the present invention, the ene reaction is preferably performed at a temperature of at least 150°C, more preferably of at least 180°C, and most preferably of at least 200°C. Preferably, the ene reaction is performed at a temperature not higher than 300°C, more preferably not higher than 250°C, and most preferably at a temperature not higher than 230°C. The reaction time preferably varies from 0.5 to 24 hours. More preferably, the reaction time lies between 2 and 14 hours. Most preferably, the reaction time lies between 5 and 9 hours. The ratio between the olefin and the cyclic anhydride of an unsaturated dicarboxylic acid preferably is between 0.8 - 2.0 to 1 (i.e. 80 to 200 mol% of olefin is used). More preferably, the ratio of olefin to cyclic anhydride of an unsaturated dicarboxylic acid is between 1.0 - 1.5 to 1 (i.e. 100-150 mol% of olefin). Most preferably, said ratio is between 1.1 - 1.3 to 1 (i.e. 110-130 mol% of olefin).

It is possible to perform the reaction between the olefinic hydrocarbons and the cyclic anhydride of an unsaturated dicarboxylic acid in the presence of an apolar organic solvent. However, this is less preferred, because the drawback to the use of solvents is that these have to be removed in a separate step afterwards. Hence, most preferably, the process is carried out in the absence of solvents. However, if the viscosity of the olefinic hydrocarbons is too high, it is recommended to add an apolar solvent such as an easily removable lower alkane.

In a preferred embodiment, step (ii) is conducted in the present of a radical scavenger. This radical scavenger serves to reduce the overall amount of by-products, in particular higher molecular weight adducts, that can be formed during step (ii), in particular olefin-anhydride-olefin adducts.

Suitable radical scavengers are hydroxyl aromatic compounds and amino aromatic compounds. Examples of such hydroxyl aromatic compounds are phenol, o-cresol, m-cresol, p-cresol, thymol, carvacrol, durenol, isodurenol, di-t-butyl hydroquinone, 2-, 3- and 4-aminophenols, hydroquinone, resorcinol, catechol, thymohydroquinone, olivetol, 4-t-butyl catechol, 2,6-di-t-butyl-4-methylphenol, and 4-methoxyphenol. Examples of suitable aminoaromatics are phenothiazine, diphenylamine, 4,4'-thio bis(6-tertiary-butyl-o-cresol), tetramethyl thiuram disulfide, 2-aminodiphenylamine, 4-aminodiphenylamine, 4,4'-diaminodiphenylamine, 2-hydroxydiphenylamine, 3-hydroxydiphenylamine, 4-hydroxydiphenylamine, di-2-tolylamine, di-3-tolylamine, di-4-tolylamine, 3,4-ditolylamine, 1-naphthylphenylamine, 2-naphthylphenylamine, 1-naphthyl-2-tolylamine, 1-naphthyl-4-tolylamine, 2-naphthyl-2-tolylamine, 2-naphthyl-4-tolylamine, and 9,10-dihydrophenazine.

More preferably, the radical scavenger is an amino aromatic compound. Most preferably, it is phenothiazine.

The cyclic anhydride of an unsaturated dicarboxylic acid according to the present invention is a compound of the general formula depicted in Figure 4, 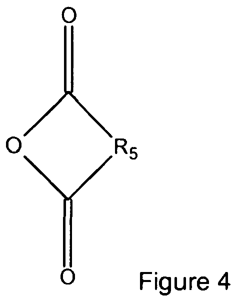 with R⁵ being an, optionally substituted, C₂ - C₄ alkenyl group. Possible substituents on the C₂ - C₄ alkenyl group include alkyl groups, alkenyl groups, aralkyl groups, or aralkenyl groups. The cyclic anhydride of an unsaturated dicarboxylic acid is preferably selected from the group consisting of maleic anhydride, itaconic anhydride, and citraconic anhydride. Most preferably, it is maleic anhydride.

The alkenyl-substituted cyclic anhydrides obtainable via the process according to the present invention, wherein olefinically unsaturated C₆ - C₂₈ hydrocarbons of which at least 30% by weight is alpha-olefin and with at least 15% by weight of said alpha-olefin being vinylidene isomer have been isomerized using a catalyst comprising an alkali metal on a carrier, are physically distinguishable from alkenyl-substituted cyclic anhydrides prepared from the same feedstock but via prior art isomerization processes. More particularly, these alkenyl-substituted cyclic anhydrides differ from alkenyl-substituted cyclic anhydrides prepared from isomerized C₆ - C₂₈ linear alpha olefins and from alkenyl-substituted cyclic anhydrides prepared from C₆ - C₂₈ unsaturated hydrocarbons the same amount of vinylidene isomer but isomerized via a prior art isomerization method, in the nature of the alkenyl chain. More particularly, they differ from each other in the nature of the substituents on this alkenyl chain. This difference in structure is for example apparent from their improved paper sizing performance.

The present invention furthermore relates to a process for the preparation of a paper sizing additive wherein a cyclic anhydride of an unsaturated dicarboxylic acid, which preferably is maleic anhydride, is reacted with one or more internal olefinically unsaturated C₆ - C₂₈ hydrocarbons, wherein said internal olefinically unsaturated C₆ - C₂₈ hydrocarbons have been prepared by subjecting one or more olefinically unsaturated C₆ - C₂₈ hydrocarbons of which at least 30% by weight is alpha-olefin to an isomerization step in the presence of a catalyst comprising an alkali metal on a carrier. The reaction conditions and preferred embodiments are as described above.

The above-described paper sizing additive is suitable for use in conjunction with paper and paperboard production in order to introduce water repellence. The alkenyl-substituted cyclic anhydride compounds are hydrophobic in character and hence they are not easily soluble in poor solvents such as water. Thus, before the sizing agent is added to a paper wet stock, the alkenyl-substituted cyclic anhydride compound is dispersed in an aqueous medium. However, since alkenyl-substituted cyclic anhydride compounds tend to decompose in the presence of water and thus lose their sizing ability, preferably, the sizing dispersions comprising alkenyl-substituted cyclic anhydride compounds are used fairly quickly subsequent to their preparation. Preferably, the sizing dispersion is formulated close to the location of intended use, i.e. at the paper mill.

In order to achieve proper sizing of paper or board, the particle size of the alkenyl-substituted cyclic anhydride compound contained in the dispersion must be below a specific value. A small particle size of the alkenyl-substituted cyclic anhydride compound is obtained by introducing high shear forces while forming the dispersion by using a high pressure unit. The preparation is performed for example in a homomixer or a homogenizer with the use of a water-soluble polymeric compound such as cationized starch or a surfactant such as polyoxyalkylene aryl ether.

The alkenyl-substituted cyclic anhydride compounds can also be used for preparing various ester, amide, imide, and other derivatives, which are used partly in applications similar to those in which actual ASA is used, for example as additives in oil and as corrosion inhibitors.

The process according to the present invention is further illustrated by the following non-limiting examples.

### EXAMPLES

### Example 1 and Comparative Example A

As the starting material were used C18 alpha-olefins ex Ineos comprising a high amount of branched starting material (about 43% by weight of vinylidene isomers).

### Example 1:

In Example 1, this starting material was isomerized using sodium alumina catalyst. In more detail, 22 g (0.159 mol) of K₂CO₃ were dissolved in 49 ml of demineralized water. Under stirring the solution was added to 100 g (0.981 mol) of γ-Al₂O₃ (*Merck,* surface 120-190 m²/g). The impregnated Al₂O₃ was dried for 3:30 h at 120°C and subsequently calcined for 3:20 h at 500°C. The carrier material was transferred afterwards into a glass vessel, which was evacuated with an oil pump and flushed with nitrogen. The carrier material was stored under nitrogen until it was used.

Subsequently, 24.97 g of the Al₂O₃, K₂CO₃ carrier material were stirred for 2 hours under vacuum (oil pump) at 160°C in a two-necked glass vessel, equipped with a magnetic stirring bar. After flushing with nitrogen, the carrier material was allowed to cool down to ambient temperature and 1.290 g (0.056 mol) sodium (Na) were added in a nitrogen stream. After complete addition of the sodium, the carrier was stirred for an additional 90 minutes at 160°C to finish the generation of the catalyst. The catalyst was obtained as a dark blue / gray powder.

The catalyst was afterwards allowed to cool down to ambient temperature and 374.87 g (1.485 mol) of a C18 alpha-olefin (ex Ineos) were added in a nitrogen stream. The reaction mixture was subsequently heated up to 62°C and kept at this temperature for 3 h. The reaction was allowed to cool down, and the isomerizate was removed from the reactor. A further workup of the isomerizate, as described in the case of iron pentacarbonyl (Fe(CO)₅) in Comparative Example A below, was not necessary.

Next, alkenyl succinic anhydride was prepared in the following manner. 126.76 g (0.502 mol) of the isomerized C18 olefin were added to the reaction vessel under a nitrogen stream. The olefin was heated up to 50°C and at this temperature 40.96 g (0.418 mol) maleic anhydride (MSA) flakes were added under a nitrogen stream. After the MSA had been melted (∼ 53°C), the reaction mixture was inertized by evacuating (oil pump) and flushing with nitrogen (three times).

Subsequently the temperature was raised to 200°C within 15 min. and after the start of the reflux of the MSA the temperature was increased to 230°C within 1:30 h.

This temperature was kept for an additional 5:30 h and afterwards the excess of olefin and the unreacted MSA were distilled at reduced pressure (oil pump). The alkenylated succinic anhydride (ASA) was obtained as a brownish clear liquid.

### Comparative Example A:

In Comparative Example A, the same starting material as used for Example 1 was isomerized using iron pentacarbonyl as the homogeneous catalyst in the same manner as described in US 4,587,374. The homogeneous catalyst and the residuals thereof, which caused an intensive dark orange-brown colourization of the isomerizate, were separated by distillation of the isomerizate under reduced pressure. This step which, due to the nature of the distillation, involves a loss of isomerized olefin (product), had to be included to prevent a negative impact on the colour of the resulting ASA.

The corresponding alkenyl succinic anhydride was prepared by adding 154.10 g (0.610 mol) of the isomerized C18 olefin and 49.85 g (0.508 mol) of maleic anhydride (MSA) flakes under a nitrogen stream to the reaction vessel. The suspension was heated up to 53°C and after the MSA had been melted, the reaction mixture was inertized by evacuating (oil pump) and flushing with nitrogen (three times). Subsequently the temperature was raised to 200°C within 15 min. and after the start of the reflux of the MSA the temperature was increased to 230°C within 1:30 h. This temperature was kept for an additional 5:30 h and afterwards the excess of olefin and the unreacted MSA were distilled at reduced pressure (oil pump). The alkenylated succinic anhydride (ASA) was obtained as a brownish clear liquid.

### Example 1 and Comparative Example A: Sizing performance:

The sizing performance of the ASA obtained according to Example 1 and Comparative Example A, respectively, was measured as Cobb 60 value (g/m²) (water absorption of the probe) as described in EN 20535 (old DIN 53132). Sizing tests were performed using a furnish of a 80/20 blend of hardwood/softwood (36° SR freeness). As filler was used 15 wt% calcium carbonate (Hydrocarb 50 BG, Omya) and the retention system was Compozil with 0.5 wt% cationic potato starch (Raisamyl 142) and 0.3 wt% silicasol Eka NP 442 (Eka Chemicals). Aluminium sulphate was used in an amount of 0.15 wt%, resulting in a pH of 7.8 in the headbox. Papers of 75 g/m² were prepared on a pilot paper machine at 2 m/min.

The ASA emulsions used as sizing agent were prepared by emulsification of 15 g ASA and 185 g starch solution (4% solids) with the aid of a kitchen blender (Osterizer). A lower Cobb means a better sizing performance and vice versa.

As can be derived from Table 1, the ASA based on the olefin from the heterogeneous isomerization has a much better performance than the ASA based on the homogeneous isomerization.

**Table 1: Cobb values for Example 1 and Comparative Example A:**

| | *Example 1* | *Comp. Example A* |
|---|---|---|
| | Na on carrier | Fe(CO)₅ |
| ASA-amount [kg/t] | (Cobb) | (Cobb) |
| | | |
| 0.6 | 33.5 | 59.7 |
| 0.9 | 24 | 28.5 |
| 1.2 | 21.7 | 24.5 |

### Examples 2-4

As the starting material, C18 alpha-olefins (ex-Chevron) comprising a low amount of branched starting material (about 8% by weight of vinylidene isomers) was used.

### Example 2:

The starting material was isomerized using sodium alumina catalyst. The Al₂O₃, K₂CO₃ carrier material of this catalyst was prepared according to the method described in Example 1. Subsequently, 26.67 g of the Al₂O₃, K₂CO₃ carrier material were stirred for 2 hours under vacuum (oil pump) at 160°C in a two necked glass vessel, equipped with a magnetic stirring bar. After flushing with nitrogen, 1.373 g (0.060 mol) sodium (Na) were added in a nitrogen stream and the carrier was stirred for an additional 90 minutes at 160°C, to finish the generation of the catalyst. The catalyst was obtained as dark blue / gray powder.

The catalyst was afterwards allowed to cool down to ambient temperature and 394.5 g (1.563 mol) of a C18 alpha-olefin were added to the catalyst in a nitrogen stream. The reaction mixture was subsequently heated up to 60°C and kept at this temperature for 3 hours. The reaction was allowed to cool down, and the isomerizate was removed from the reactor. A further workup of the isomerizate was not necessary.

Next, alkenyl succinic anhydride was prepared in the following manner. 123.72 g (0.490 mol) of the isomerized C18 olefin were added to the reaction vessel under a nitrogen stream. The olefin was heated up to 50°C and at this temperature 40.04 g (0.408 mol) maleic anhydride (MSA) flakes were added under a nitrogen stream. After the MSA had been melted (∼53°C), the reaction mixture was inertized by evacuating (oil pump) and flushing with nitrogen (three times). Subsequently the temperature was raised to 200°C within 15 min. and after the start of the reflux of the MSA the temperature was increased to 230°C within 90 minutes.

This temperature was kept for an additional 5:30 h and afterwards the excess of olefin and the unreacted MSA were distilled at reduced pressure (oil pump). The alkenylated succinic anhydride (ASA) was obtained as a brownish clear liquid.

### Example 3

In Example 3 the starting material was isomerized as described in Example 2, using the sodium alumina catalyst.

Next, alkenyl succinic anhydride was prepared in the following manner. 125.87 g (0.499 mol) of isomerized C18 olefin were added to the reaction vessel. Then the olefin was inertized by evacuating (oil pump) and flushing with nitrogen (three times). Subsequently the olefin was heated to the reaction temperature of 230°C. When the reaction temperature was reached, 40.74 g (0.415 mol) of molten MSA were added dropwise within two hours to the olefin. After complete addition of the MSA, the temperature of 230°C was kept for additional 5 hours. Afterwards the excess of olefin and the unreacted MSA were distilled under reduced pressure. The alkenylated succinic anhydride (ASA) was obtained as a brownish clear liquid.

### Example 4

In Example 4 the starting material was isomerized as described in Example 2, using the sodium alumina catalyst.

Next, alkenyl succinic anhydride was prepared in the following manner. 111.10 g (0.440 mol) of isomerized C18 olefin were added to the reaction vessel. Subsequently 6.5 mg of phenothiazine (0.033 * 10⁻³ mol, 0.009 mol% with respect to the molar amount of MSA) were added to the olefin. Then the olefin / phenothiazine mixture was inertized by evacuating (oil pump) and flushing with nitrogen (three times). Subsequently the mixture was heated to the reaction temperature of 230°C. When the reaction temperature was reached, 35.96 g (0.367 mol) of molten MSA were added dropwise within two hours to the mixture. After complete addition of the MSA, the temperature of 230°C was kept for additional five hours. Afterwards the excess of olefin and the unreacted MSA were distilled under reduced pressure. The alkenylated succinic anhydride (ASA) was obtained as a brownish clear liquid.

Analysis of the products of the above Examples with gel permeation chromatography showed (see Table 2) that the addition of phenothiazine resulted in a reduction of the amount of by-products, in particular the amount of olefin-maleic anhydride-olefin adduct (OMO).

**Table 2: Content of the olefin - anhydride - olefin adduct (adduct 1)**

| experiment | Example 1 | Comparative Example A | Example 2 | Example 3 | Example 4 |
|---|---|---|---|---|---|
| scavenger [mol-%] | - | - | - | - | phenothiazine 0.009 |
| OMO [%-GPC] | 5.6 | 4.5 | 3.3 | 7.5 | 1.5 |

## Claims

1. Process for the preparation of an alkenyl-substituted cyclic anhydride compound comprising the steps of
(i) subjecting one or more olefinically unsaturated C₆ - C₂₈ hydrocarbons of which at least 30% by weight is alpha-olefin and with at least 15%, preferably at least 25% by weight of said alpha-olefin being vinylidene isomer to a double bond isomerization step by contacting them with a catalyst comprising an alkali metal on a carrier, and
(ii) reacting the resulting isomerized olefinic C₆ - C₂₈ hydrocarbons with a cyclic anhydride of an unsaturated dicarboxylic acid to form the alkenyl-substituted cyclic anhydride compound.

2. Process according to claim 1 wherein the cyclic anhydride of an unsaturated dicarboxylic acid is selected from the group consisting of maleic anhydride, itaconic anhydride, and citraconic anhydride.

3. Process according to any one of the preceding claims wherein a radical scavenger is present during step (ii), said radical scavenger being selected from hydroxyl aromatic compounds and amino aromatic compounds.

4. Process according to any one of the preceding claims wherein the olefinically unsaturated hydrocarbon is an olefinically unsaturated C₁₄ - C₂₄ hydrocarbon, preferably an olefinically unsaturated C₁₆ - C₂₂ hydrocarbon, most preferably an olefinically unsaturated C₁₆ or C₁₈ hydrocarbon or a mixture of olefinically unsaturated C₁₆ and C₁₈ hydrocarbons.

5. Process according to any one of the preceding claims wherein the catalyst comprises as the alkali metal sodium or potassium, either alone or in combination with a compound of the other metal.

6. Process according to any one of the preceding claims wherein the carrier is selected from the group consisting of aluminium oxide, silicon oxide, magnesium oxide, silicon oxide-aluminium oxide, silicon oxide-aluminium oxide, silicon oxide-aluminium oxide-magnesium oxide, titanium oxide, zirconium oxide, bauxite, clays, pumice, activated carbon, and molecular sieves.

7. Process according to any one of the preceding claims wherein the alkali metal is present in the catalyst in an amount of 1 - 30% by weight, based on the weight of the carrier.

8. Process according to any one of the preceding claims wherein the alumina carrier is gamma-alumina, preferably with a specific surface of at least 100 m²/g_{.}

9. Process according to any one of the preceding claims wherein the isomerization step is carried out under an inert atmosphere at a temperature of between 10 and 200°C, preferably between 20 and 60°C.

10. Process according to any one of the preceding claims wherein step (ii) is performed at a temperature of between 150°C and 300°C, and wherein the ratio between the olefin and the cyclic anhydride of an unsaturated dicarboxylic acid is between 0.8 - 2.0 to 1.

11. Use of an alkenyl-substituted cyclic anhydride obtainable by the process of any one of claims 1-10 as a paper sizing additive in conjunction with paper and paperboard production.

12. Process for the preparation of a paper sizing additive wherein a cyclic anhydride of an unsaturated dicarboxylic acid, preferably maleic anhydride, is reacted with one or more internal olefinically unsaturated C₆ - C₂₈ hydrocarbons, wherein said internal olefinically unsaturated C₆ - C₂₈ hydrocarbons have been prepared by subjecting one or more olefinically unsaturated C₆ - C₂₈ hydrocarbons of which at least 30% by weight is alpha-olefin and with at least 15%, preferably at least 25% by weight of said alpha-olefin being vinylidene isomer to an isomerization step in the presence of a catalyst comprising an alkali metal on a carrier.

13. Process according to claim 12 wherein the catalyst comprises as the alkali metal sodium or potassium, either alone or in combination with a compound of the other metal, and the carrier is selected from the group consisting of aluminium oxide (gamma-, eta-, or theta-), silicon oxide, magnesium oxide, silicon oxide-aluminium oxide, silicon oxide-aluminium oxide-magnesium oxide, titanium oxide, zirconium oxide, bauxite, clays, pumice, activated carbon, and molecular sieves.

## Patentansprüche

1. Verfahren zur Herstellung einer alkenylsubstituierten cyclischen Anhydridverbindung umfassend die Schritte
(i) Unterwerfen eines oder mehrerer olefinisch ungesättigter C₆-C₂₈-Kohlenwasserstoffe, von denen mindestens 30 Gewichtsprozent alpha-Olefin und mindestens 15 Gewichtsprozent, vorzugsweise mindestens 25 Gewichtsprozent des alpha-Olefins ein Vinylidenisomer ist, einem Doppelbindungs-Isomerisierungsschritt durch in Kontakt bringen mit einem Katalysator, umfassend ein Alkalimetal auf einem Träger und
(ii) Reagieren der resultierenden isomerisierten olefinischen C₆-C₂₈-Kohlenwasserstoffe mit einem cyclischen Anhydrid einer ungesättigten Dicarbonsäure, um die alkenylsubstituierte cyclische Anhydridverbindung zu bilden.

2. Verfahren gemäß Anspruch 1, wobei das cyclische Anhydrid einer ungesättigten Dicarbonsäure ausgewählt ist aus der Gruppe bestehend aus Maleinsäureanhydrid, Itaconsäureanhydrid und Citraconsäureanhydrid.

3. Verfahren gemäß einem der vorstehenden Ansprüche, wobei ein Radikalfänger während des Schrittes (ii) anwesend ist, welcher ausgewählt ist aus hydroxylaromatischen Verbindungen und aminoaromatischen Verbindungen.

4. Verfahren gemäß einem der vorstehenden Ansprüche, wobei der olefinisch ungesättigte Kohlenwasserstoff ein olefinisch ungesättigter C₁₄-C₂₄-Kohlenwasserstoff, bevorzugt ein olefinisch ungesättigter C₁₆-C₂₂-Kohlenwasserstoff, am stärksten bevorzugt ein olefinisch ungesättigter C₁₆- oder C₁₈-Kohlenwasserstoff oder ein Gemisch von olefinisch ungesättigten C₁₆- und C₁₈-Kohlenwasserstoffen ist.

5. Verfahren gemäß einem der vorstehenden Ansprüche, wobei der Katalysator als Alkalimetall Natrium oder Kalium umfasst, entweder allein oder in Kombination mit einer Verbindung des anderen Metalls.

6. Verfahren gemäß einem der vorstehenden Ansprüche, wobei der Träger aus der Gruppe bestehend aus Aluminiumoxid, Siliciumoxid, Magnesiumoxid, Siliciumoxid-Aluminiumoxid, Siliciumoxid-Aluminiumoxid-Magnesiumoxid, Titanoxid, Zirkoniumoxid, Bauxit, Tonen, Bimsstein, Aktivkohle und Molekularsieben ausgewählt ist.

7. Verfahren gemäß einem der vorstehenden Ansprüche, wobei das Alkalimetall im Katalysator in einer Menge von 1-30 Gewichtsprozent bezogen auf das Gewicht des Trägers vorliegt.

8. Verfahren gemäß einem der vorstehenden Ansprüche, wobei der Aluminiumträger gamma-Aluminiumoxid ist, vorzugsweise mit einer spezifischen Oberfläche von mindestens 100 m²/g.

9. Verfahren gemäß einem der vorstehenden Ansprüche, wobei der Isomerisierungsschritt unter einer inerten Atmosphäre bei einer Temperatur zwischen 10 und 200°C, vorzugsweise zwischen 20 und 60°C durchgeführt wird.

10. Verfahren gemäß einem der vorstehenden Ansprüche, wobei Schritt (ii) bei einer Temperatur zwischen 150°C und 300°C durchgeführt wird und wobei das Verhältnis zwischen dem Olefin und dem cyclischen Anhydrid einer ungesättigten Dicarbonsäure zwischen 0,8 - 2,0 zu 1 beträgt.

11. Verwendung eines alkenylsubstituierten cyclischen Anhydrids erhältlich durch das Verfahren gemäß einem der Ansprüche 1 bis 10 als Papierleimungszusatz in Verbindung mit Papier- und Pappeherstellung.

12. Verfahren zur Herstellung eines Papierleimungszusatzes, wobei ein cyclisches Anhydrid einer ungesättigten Dicarbonsäure, vorzugsweise Maleinsäureanhydrid, mit einem oder mehreren intern olfinisch ungesättigten C₆-C₂₈-Kohlenwasserstoffen umgesetzt wird, wobei die intern olefinisch ungesättigten C₆-C₂₈-Kohlenwasserstoffe durch Unterwerfen eines oder mehrerer olefinisch ungesättigter C₆-C₂₈-Kohlenwasserstoffe, von denen mindestens 30 Gewichtsprozent alpha-Olefin und mindestens 15 Gewichtsprozent, vorzugsweise mindestens 25 Gewichtsprozent des alpha-Olefins ein Vinylidenisomer ist, einem Isomerisierungsschritt in Gegenwart eines Katalysators, umfassend ein Alkalimetal auf einem Träger, hergestellt wurden.

13. Verfahren gemäß Anspruch 12, wobei der Katalysator als Alkalimetall Natrium oder Kalium umfasst, entweder allein oder in Kombination mit einer Verbindung des anderen Metalls, und der Träger aus der Gruppe bestehend aus Aluminiumoxid (gamma-, eta-oder theta-), Siliciumoxid, Magnesiumoxid, Siliciumoxid-Aluminiumoxid, Siliciumoxid-Aluminiumoxid-Magnesiumoxid, Titanoxid, Zirkoniumoxid, Bauxit, Tonen, Bimsstein, Aktivkohle und Molekularsieben ausgewählt ist.

## Revendications

1. Procédé pour la préparation d'un composé anhydride cyclique substitué en alcényle comprenant les étapes consistant à :
(i) soumettre un ou plusieurs hydrocarbures en C₆-C₂₈ oléfiniquement insaturés dont au moins 30% en poids sont une alpha-oléfine et avec au moins 15%, de préférence au moins 25% en poids de ladite alpha-oléfine étant un isomère de vinylidène à une étape d'isomérisation à double liaison, en les mettant en contact avec un catalyseur comprenant un métal alcalin sur un support, et
(ii) faire réagir les hydrocarbures en C₆-C₂₈ oléfiniques isomérisés obtenus avec un anhydride cyclique d'un acide dicarboxylique insaturé pour former le composé anhydride cyclique substitué en alcényle.

2. Procédé selon la revendication 1, dans lequel l'anhydride cyclique d'un acide dicarboxylique insaturé est choisi dans le groupe constitué d'anhydride maléique, d'anhydride itaconique et d'anhydride citraconique.

3. Procédé selon l'une quelconque des revendications précédentes, dans lequel un inhibiteur de radicaux est présent pendant l'étape (ii), ledit inhibiteur de radicaux étant choisi parmi des composés aromatiques hydroxyle et des composés aromatiques amino.

4. Procédé selon l'une quelconque des revendications précédentes, dans lequel l'hydrocarbure oléfiniquement insaturé est un hydrocarbure en C₁₄-C₂₄ oléfiniquement insaturé, de préférence un hydrocarbure en C₁₆-C₂₂ oléfiniquement insaturé, de manière davantage préférée un hydrocarbure en C₁₆ ou C₁₈ oléfiniquement insaturé ou un mélange d'hydrocarbures en C₁₆ et C₁₈ oléfiniquement insaturés.

5. Procédé selon l'une quelconque des revendications précédentes, dans lequel le catalyseur comprend, comme métal alcalin, le sodium ou le potassium, soit seul ou en combinaison avec un composé de l'autre métal.

6. Procédé selon l'une quelconque des revendications précédentes, dans lequel le support est choisi dans le groupe constitué d'oxyde d'aluminium, d'oxyde de silicium, d'oxyde de magnésium, d'oxyde de silicium-oxyde d'aluminium, d'oxyde de silicium-oxyde d'aluminium-oxyde de magnésium, d'oxyde de titane, d'oxyde de zirconium, de bauxite, d'argiles, de pierre ponce, de charbon actif, et de tamis moléculaires.

7. Procédé selon l'une quelconque des revendications précédentes, dans lequel le métal alcalin est présent dans le catalyseur dans une quantité de 1-30% en poids, sur la base du poids du support.

8. Procédé selon l'une quelconque des revendications précédentes, dans lequel le support d'alumine est une gamma-alumine, de préférence avec une surface spécifique d'au moins 100 m²/g.

9. Procédé selon l'une quelconque des revendications précédentes, dans lequel l'étape d'isomérisation est réalisée sous une atmosphère inerte à une température comprise entre 10 et 200°C, de préférence entre 20 et 60°C.

10. Procédé selon l'une quelconque des revendications précédentes, dans lequel l'étape (ii) est réalisée à une température comprise entre 150°C et 300°C, et dans lequel le rapport entre l'oléfine et l'anhydride cyclique d'un acide dicarboxylique insaturé est compris entre 0,8 -2,0 et 1.

11. Utilisation d'un anhydride cyclique substitué en alcényle pouvant être obtenu par le procédé selon l'une quelconque des revendications 1-10, comme un additif d'encollage de papier, en combinaison avec la production de papier et de carton.

12. Procédé pour la préparation d'un additif d'encollage de papier, dans lequel un anhydride cyclique d'un acide dicarboxylique insaturé, de préférence l'anhydride maléique, est mis en réaction avec un ou plusieurs hydrocarbures en C₆-C₂₈ oléfiniquement insaturés internes, dans lequel lesdits hydrocarbures en C₆-C₂₈ oléfiniquement insaturés internes ont été préparés en soumettant un ou plusieurs hydrocarbures en C₆-C₂₈ oléfiniquement insaturés, dont au moins 30% en poids sont une alpha-oléfine et avec au moins 15%, de préférence au moins 25% en poids de ladite alpha-oléfine étant un isomère de vinylidène à une étape d'isomérisation en présence d'un catalyseur comprenant un métal alcalin sur un support.

13. Procédé selon la revendication 12, dans lequel le catalyseur comprend, comme métal alcalin, le sodium ou le potassium, soit seul ou en combinaison avec un composé de l'autre métal, et le support est choisi dans le groupe constitué d'oxyde d'aluminium (gamma-, êta- ou thêta-), l'oxyde de silicium, l'oxyde de magnésium, l'oxyde de silicium-oxyde d'aluminium, l'oxyde de silicium-oxyde d'aluminium-oxyde de magnésium, l'oxyde de titane, l'oxyde de zirconium, la bauxite, les argiles, la pierre ponce, le charbon actif et les tamis moléculaires.
